# EUROPÄISCHE PATENTSCHRIFT

(11) **EP 3 601 411 B1**
(45) Veröffentlichungstag und Bekanntmachung des Hinweises auf die Patenterteilung: **03.04.2024**
(21) Anmeldenummer: 18724772.1
(22) Anmeldetag: 23.03.2018
(51) Int. Cl.: C08G 69/48, C13K 11/00, C07H 3/02, C08G 73/02

(54) **KATIONISCHE POLYMERE MIT D-FRUCTOSE-SUBSTITUENTEN**
CATIONIC POLYMERS WITH D-FRUCTOSE SUBSTITUENTS
POLYMÈRES CATIONIQUES À SUBSTITUANTS DE D-FRUCTOSE

(30) Priorität: 23.03.2017 DE 102017003004
(43) Veröffentlichungstag der Anmeldung: 05.02.2020
(73) Patentinhaber: SmartDyeLivery GmbH, 07743 Jena (DE)
(72) Erfinder: GOTTSCHALDT, Michael, 07751 Beutnitz (DE); PRÖHL, Michael, 38179 Schwülper (DE); ENGLERT, Christoph, 07745 Jena (DE); SCHUBERT, Ulrich Sigmar, 07743 Jena (DE)
(74) Vertreter: Donath, Dirk
(86) Internationale Anmeldenummer: PCT/DE2018/100268
(87) Internationale Veröffentlichungsnummer: WO 2018/171845

(56) Entgegenhaltungen:
- WO-A2-03/008555
- US-A1- 2006 093 674

## Beschreibung

Die Erfindung betrifft neue kationische Polymere, die mit D-Fructose-konjugiert sind, wodurch sie selektiv mit spezifischen Strukturelementen an Zelloberflächen wechselwirken können.

Bekannte kationische Polymere, wie Poly(ethylenimin) (PEI) oder Poly(L-Lysin) (PLL), weisen wesentliche Nachteile auf (H. Lv et al. (2006): "Toxicity of cationic lipids and cationic polymers in gene delivery" Journal of Controlled Release 114: 100-109):
- allgemeine und unspezifische Zytotoxizität gegenüber Zellen,
- Auslösen von Aggregation und Hämolyse von Blutzellen.

Kationische Polymere weisen aufgrund ihrer hohen Dichte an positiven Ladungen die Fähigkeit auf, negativ geladenes, genetisches Material, wie beispielsweise siRNA (small interfering ribonucleic acid) oder pDNA (plasmid desoxyribonucleic acid), zu komplexieren. Die so entstehenden Addukte zwischen kationischen Polymeren und genetischem Material werden Polyplexe genannt, mit deren Hilfe der Transport von genetischem Material (z.B. siRNA) in Zellen erreicht werden kann.

Kationische Polymere konnten bereits erfolgreich als Zusatzstoff bei Zuckertensiden zur Verbesserung der sensorischen Eigenschaften und des Schaumgefühls bei kosmetischen Anwendungen eingesetzt werden (Patentanmeldung: Verwendung von kationischen Biopolymeren zur Verbesserung der sensorischen Eigenschaften zuckertensidhaltiger Zubereitungen, DE19605355A1, Joerg Kahre, Rolf Wachter).

Weiterhin können kationische Polymere mit kleinen Proteinsequenzen (z.B.: RGD Peptide) funktionalisiert und damit die Selektivität beeinflusst werden (C. L. Waite et al. (2009): "PAMAM-RGD Conjugates Enhance siRNA Delivery Through a Multicellular Spheroid Model of Malignant Glioma" Bioconjugate Chemistry: 20: 1908-1916). Der genaue Mechanismus ist hierbei unklar und das Vorhandensein von Integrin (Transportmembranprotein in tierischen Zellen) ist notwendig.

Eine kovalente Verknüpfung von kationischen Polymeren mit Zuckern ist ebenfalls bekannt geworden.

Poly(propylenimin)-Polymere konnten mit D-Mannose funktionalisiert und deren Eigenschaften im erfolgreichen Einsatz gegen HIV untersucht werden. Dabei dient dieser Ansatz ausschließlich für das gezielte Ansteuern von Immunzellen, sogenannten Makrophagen, mit Lectin-Rezeptoren. (T. Dutta et. al (2007): "Targeting potential and anti-HIV activity of lamivudine loaded mannosylated poly (propyleneimine) dendrimer" Biochimica et Biophysica Acta (BBA) - General Subjects: 1770, 681-686).

WO 2003/008555 A2 offenbart ein polymeres Konjugat, das als Genträger fungiert und zur gezielten Genabgabe verwendet werden kann, umfassend ein kationisches Polymer (CP)-Element, auf das ein Poly(ethylenglykol)-Element (PEG) aufgepfropft ist, das wiederum kovalent an eine Targeting-Einheit (TM) gebunden ist, wobei 0. 1 bis 10 Molprozent der kationischen Gruppen des CP mit dem TM-PEG substituiert sind, und wobei das polymere Konjugat durch ein Verfahren synthetisiert wird, das umfasst:
1) Erhalten eines TM mit einer Amingruppe und einem bifunktionellen PEG, das ein λ-Vinylsulfon (VS) an einem Ende und einen ω-N-Hydroxysuccinimidylester (NHS) am anderen Ende enthält (VS-PEG-NHS);
2) Umsetzen der Amingruppe des TM mit der VS-Gruppe des VS-PEG-NHS, um VS-PEG-TM zu erhalten;

und 3) Umsetzen der VS-Gruppe des VS-PEG-TM mit den Amingruppen des CP bei einem pH-Wert von 4 bis 9,
wobei das TM aus der Gruppe ausgewählt ist, die aus Monosacchariden, Oligosacchariden, terminalen Zuckerresten, Polysacchariden, terminalen Monoglycosiden, Targeting-Peptiden und Antikörpern besteht oder die aus Galaktose-, Laktose-, Fruktose- und Mannose-terminalen Monoglykosiden besteht und wobei das PA Polyethylenimin (PEI) ist.

Der Nachteil dieser Synthese von D-Galactose-konjugierten Poly(ethylenglycol)-poly(ethylenimin)-Copolymeren für die Transfektion genetischen Materials in Hepatozyten besteht darin, dass dieser Ansatz nur für Leberzellen mit ASGP-Rezeptoren geeignet ist [Publikation: K. Sagara et al. (2002). "A new synthesis of galactose-poly(ethylene glycol)-polyethylenimine for gene delivery to hepatocytes" Journal of Controlled Release 79(1-3): 271-281)].

Lactose und α-Cyclodextrin wurden an ein kationisches, sternförmiges Poly(amidoamine) (PAMAM) Dendrimer gekoppelt zur Behandlung von familiärer, amyloidotischer Polyneuropathie. Dabei zielt der Ansatz lediglich auf die Transthyretin-Genexpression in Hepatozyten ab (Y. Hayashi et. al (2012): "Potential Use of Lactosylated Dendrimer (G3)/α-Cyclodextrin Conjugates as Hepatocyte-Specific siRNA Carriers for the Treatment of Familial Amyloidotic Polyneuropathy" Molecular Pharmaceutics: 9, 1645-1653).

Kationische Liposome wurden mit D-Fucose modifiziert und auf ihren Einfluss auf Adenovirus-induzierte Immunantworten untersucht. Dieser Ansatz dient ausschließlich der gezielten Lieferung des spezifischen Transkriptionsfaktors NF-κB zu Milz- und Lebermakrophagen. (H. Huang et al. (2009): "Suppressive effects of sugar-modified cationic liposome/NF-κB decoy complexes on adenovirus vector-induced innate immune responses" Journal of Controlled Release: 133, 139-145).

Acrylat- bzw. Methacrylat-basierte kationische Polymere mit glykosidisch gebundenen Sacchariden wurden beschrieben. Dabei beschreibt der Ansatz lediglich die chemische Zusammensetzung solcher Polymere und erwähnt in keinerlei Hinsicht eine mögliche biologische Anwendung. Mit den dort beschriebenen, glykosidisch gebundenen ZuckerResten kann eine Wechselwirkung mit Zucker-Transportern in Zellmembranen auch nicht erreicht werden (Patentanmeldung: Novel Glycopolymers, Uses Thereof, and Monomers Useful for Preparation Thereof, US20080281064A1, Stephanie Chiron, Marie-Pierre LaBeau, Etienne Fleury, David Viet, Sylvain Cottaz, Hugues Driguez, Sami Halila).

Nucleinsäuren und deren Polyplexe mit kationischen Polymeren wurden beschrieben. Bei diesem Ansatz waren Zucker-Moleküle bei der Polyplex-Bildung in Lösung anwesend, sind jedoch nicht kovalent am kationischen Polymer gebunden. Damit erfüllen sie keine direkten Targeting-Funktionen. (Patentanmeldung: Nucleic acid-cationic polymer compositions and methods of making and using the same, WO2016178233A1, Abraham Hochberg, Jennifer Gallula).

Der Erfindung liegt die Aufgabe zugrunde, neuartige, biokompatible, einfach herstellbare, D-Fructose-konjugierte kationische Polymere zu schaffen, die eine erhöhte Selektivität gegenüber bestimmten Zellarten aufweisen.

Dabei bezieht sich der Begriff Selektivität zum einen auf die Wechselwirkung der neuartigen D-Fructose-konjugierten, kationischen Polymeren mit bestimmten Strukturelementen an der Zelloberfläche und zum anderen auf die zytotoxische Wirkung auf bestimmte Zelltypen. Hierbei ist als nichtbeschränkendes Beispiel insbesondere die selektive, zytotoxische Wirkung auf GLUT5-überexprimierende Zelltypen, wie z.B. ein Großteil der Brustkrebszelltypen, von Interesse.

Erfindungsgemäß werden zur Lösung dieser Aufgabe kationische Polymere mit D-Fructose-Substituenten gefunden, die aus einer Grundstruktur der allgemeinen Formel (I) bestehen:

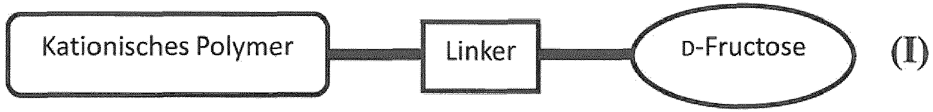

Kationisches Polymer ist dabei eine makromolekulare Verbindung aus n Wiederholungseinheiten (bevorzugt n = 10 bis 1000) mit einer oder mehreren positiven Ladungen.

Nichtbeschränkende Beispiele für bevorzugte kationische Polymere können hierbei Poly-L-Lysin (PLL), Polyethylenimin (PEI) oder Dextrane, wie Diethylaminoethyl-Dextran (DEAE-D) oder Dextran-Spermin (D-SPM) oder Polymethacrylate, wie Poly-(2-dimethylamino)ethyl-methacrylat (PDMAEMA) und Poly-dimethyl-aminoethylmethacrylat (PDAMA) sein.

Linker sind hierbei ein oder mehrere Atome oder funktionelle Gruppen, die das kationische Polymer mit der D-Fructose-Einheit verbinden. Geeignet dafür sind z.B. ein beliebiger Alkyl- oder Aryl-Rest, ein beliebiger Alkenyl oder Alkinyl-Rest, ein Ether oder Thioether, ein Amin, ein Ester-, Amid- oder ein anderes Carbonsäure-Derivat, ein Heterozyklus (z.B. Triazol oder Maleimid), ein Disulfid, ein Imin bzw. ein Imid.

Als D-Fructose und seine Derivate werden alle chemischen Moleküle verstanden, die auf D-Fructose basieren und dabei die Stereochemie der Positionen 3, 4 und 5 in offener oder geschlossener Form beibehalten. Chemische Modifikationen, insbesondere das Einführen von funktionellen Gruppen, wie z.B. Thiol-, Azid-, Carbonsäuren- und ihre Derivate und / oder Amino-Gruppen an eine oder mehrere Positionen des Zuckers (aber nicht am glykosidischen C2-Atom der D-Fructose), unter Beibehaltung der Stereochemie der Positionen 3, 4 und 5 werden hierbei ebenfalls als D-Fructose-Derivate verstanden.

Die D-Fructose besitzt eine Keto-Funktionalität in offenkettiger Form, was die chemischen Eigenschaften gänzlich verändert. Sie wird, analog zu anderen Zuckern, über spezifische Transportproteine (GLUTs) in die Zellen eingeschleust und verstoffwechselt. Der für D-Fructose verantwortliche Transporter ist der GLUT5-Transporter (A. Godoy et al. (2006): "Differential subcellular distribution of glucose transporters GLUT1-6 and GLUT9 in human cancer: Ultrastructural localization of GLUT1 and GLUT5 in breast tumor tissues." Journal of Cellular Physiology 207 (3): 614-627).

Überraschenderweise hat sich das D-Fructose-substituierte, kationische Polymer **P3** als vorteilhaft gezeigt:

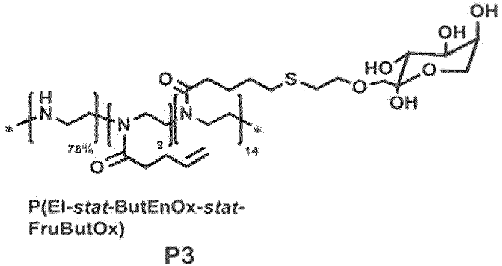

Im Vergleich zu unmodifizierten, kationischen Polymeren (z.B. L-PEI) besitzt **P3:**
- eine erhöhte Wasserlöslichkeit;
- Zytotoxizität gegenüber Brustkrebszellen, wie z.B. MDA-MB-231;
- keine Zytotoxizität gegenüber Nichtkrebszellen, wie z.B. HUVEC oder L929;
- stark verringerte hämolytische Aktivität gegenüber Blutzellen;
- keine Auslösung der Aggregation von Blutbestandteilen;
- sowie die Fähigkeit, Polyplexe mit negativ geladenen Biomolekülen, wie beispielsweise pDNA oder siRNA, zu bilden.

Die Erfindung soll nachstehend anhand der Synthese von D-Fructose-konjugierten, kationischen Polymeren (basierend auf linearem Poly(ethylenimin) (L-PEI, (I)) und verzweigtem Poly(ethylenimin) (B-PEI, (II)) gezeigt werden.

### (I) Synthese von D-Fructose-konjugiertem (unverzweigtem) L-PEI

### 1. Synthese des SH-funktionalisierten D-Fructose-Derivats in einer vierstufigen Synthese

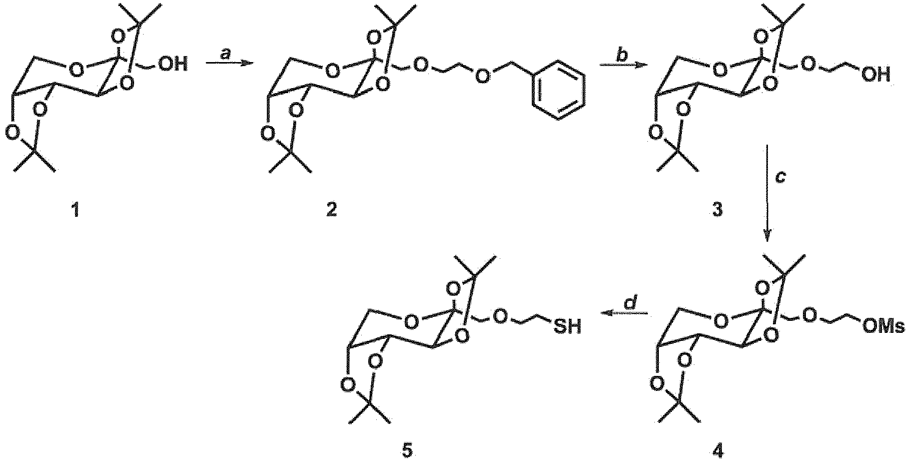

Schematische Darstellung der vierstufigen Synthese von 1-*O*-(2-Mercapto-ethyl)-2,3:4,5-di-*O*-isopropylidene-β-D-fructopyranosid: a) Benzyl-2-bromoethyl-ether, NaH, THF, rt; b) H₂/Pd (C), CH₃OH, rt; c) Mesylchlorid, Et₃N, 4-DMAP, CH₂Cl₂, 0 °C; d) 1. Thioharnstoff, Butanon, 95 °C, 2. K₂S₂O₅, CH₂Cl₂ / H₂O, 50 °C.

Das D-Fructose-Derivat **5** wurde vollständig charakterisiert und alle einzelnen Schritte konnten in hohen Ausbeuten durchgeführt werden. Die Einführung des Thiols dient dem Anhängen des Zuckers an das Polymer *via* photokatalysierter Thiol-En-Click-Reaktion.

### 2. Synthese des Block-Co-Polymers mit anschließendem Thiol-En-Click zwischen D-Fructose und Polymer-Precursor und Entschützung der Zucker-Einheit

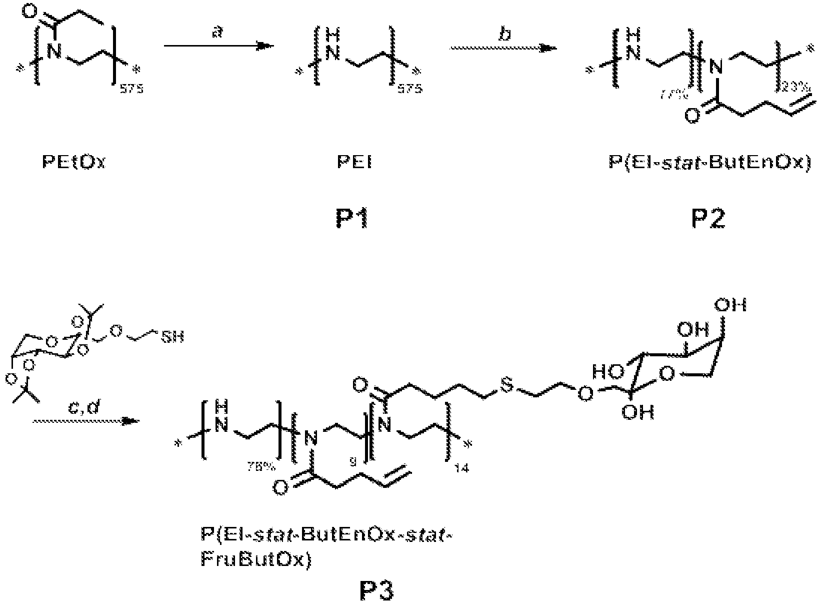

Schematische Darstellung der Synthese von P(EI-stat-ButEnOx-*stat*-FruButOx): a) 6 M HCl, 100 °C, Rückfluss; b) Pyridin, 4-DMAP, 80 °C; c) D-Fructose-Derivat **(5),** Methanol, 2,2-Dimethoxy-2-phenylacetophenon, 25 °C, UV = 365 nm; d) THF/H₂O, 2M HCl, 40 °C.

Vorliegende Copolymere und entsprechende Zwischenstufen konnten umfangreich charakterisiert werden. Als Precursor diente ein Copolymer bestehend aus Ethylenimin (EI) und mit Doppelbindungen funktionalisiertes EI. Im letzten Schritt erfolgte das Anbringen des Zucker-Derivats **5** *via* photokatalysierter Thiol-En-Click-Reaktion. Saure Entschützung resultierte im wasserlöslichen Polymer **P3.**

### (II) Die Synthese von D-Fructose konjugiertem, verzweigtem Poly(ethylenimin) (B-PEI)

### 1. Synthese von Epoxy-funktionalisierter D-Fructose

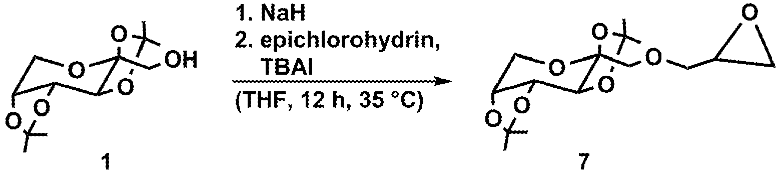

Ausgehend von kommerziell verfügbarer, Isopropyliden-geschützter D-Fructose kann durch eine Williamson-Veretherung mit Epichlorhydrin, die Epoxy-funktionalisierte D-Fructose hergestellt werden.

### 2. Kopplung von Epoxy-funktionalisierter D-Fructose mit (verzweigtem) B-PEI

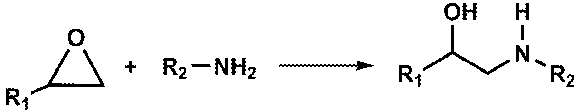

Schematische Darstellung der allgemeinen Ringöffnungsreaktion zwischen Epoxiden und primären Aminen.

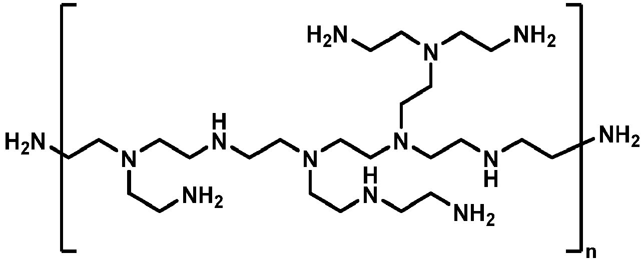

Schematische Darstellung einer möglichen Wiederholungseinheit von verzweigtem Poly(ethylenimin) (B-PEI).

Durch dreitägiges Rühren bei Raumtemperatur in Methanol kann B-PEI durch eine Ringöffnungsreaktion mit dem vorher synthetisierten D-Fructose-Derivat funktionalisiert werden. Es wurden D-Fructose konjugierte B-PEIs mit 14%, 23%, 28%, 39% und 76% funktionalisierten, primären Aminogruppen hergestellt.

### 3. Abspalten der Schutzgruppen an den Fructose-Resten

Die saure Abspaltung der Isopropyliden-Schutzgruppen in Anwesenheit von Wasser erfolgte nach mehrtägiger Erwärmung der kationischen Polymere mit gebundenen D-Fructose-Derivaten bei 40 °C unter Verwendung von 2M HCl. Dialysen (Celluloseester, MWCO: 500-1000 Da) gegen Wasser resultierten in D-Fructose funktionalisierten B-PEIs.

Das Polymer **P3** wurde einer intensiven, biologischen Evaluation unterworfen.

### a) Zytotoxizität und Hämokompatibilität

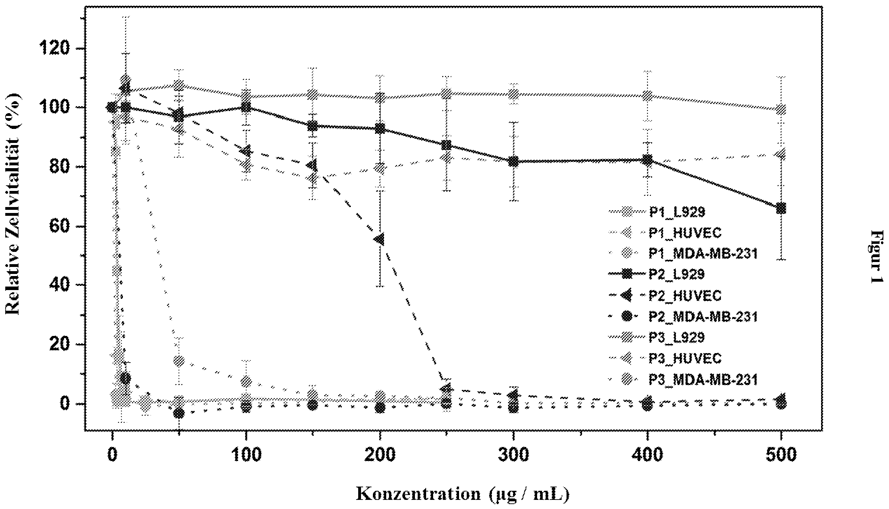

Figur **1** zeigt Zelltyp-abhängige Zytotoxizitätsstudien mittels alamarBlue-Assay der Polymere **P1, P2** und **P3.** Unbehandelte Zellen dienten als Referenz für 100%ige Vitalität. Die Zellen wurden 24 h mit den angegebenen Polymerkonzentrationen behandelt.

Überraschenderweise zeigte das D-Fructose-konjugierte Polymer **P3** eine erhöhte Toxizität gegenüber der Brustkrebszelllinie MDA-MB-231, während Nicht-Krebszellen (HUVEC und L929) keine signifikante Verringerung der Zellvitalität zeigten. Polymere **P2** und **P1** zeigten keinerlei Selektivität (Figur 1).

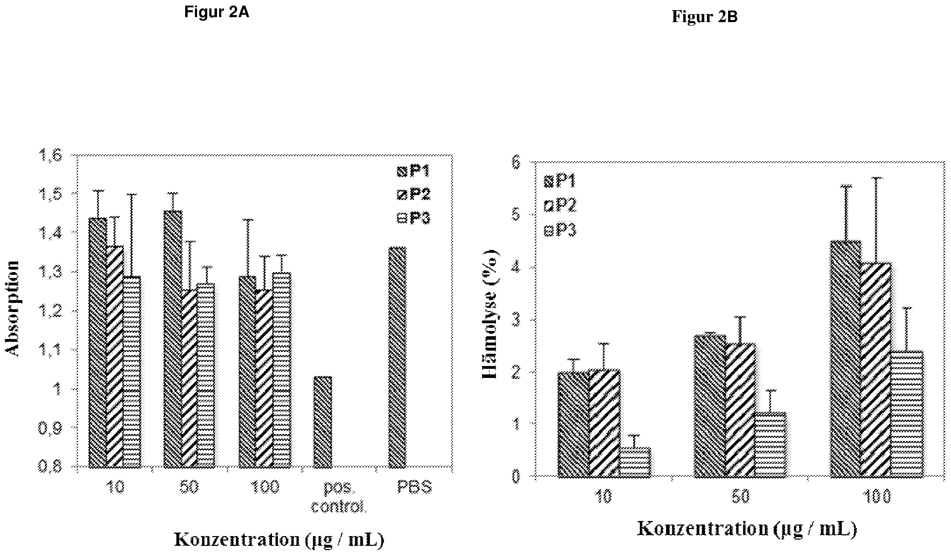

Figur 2A zeigt den Erythrozyt-Aggregations-Assay der Polymere mit angegebenen Konzentrationen. B-PEI diente als Positivkontrolle, PBS als Negativkontrolle. Figur 2B zeigt den Hämolyse-Assay der Erythrozyten nach Inkubation mit den Polymeren mit den angegebenen Konzentrationen. Triton X-100 diente als Positivkontrolle (100 % Hämolyse) und PBS als Negativkontrolle (1.99 %). Ein Wert kleiner als 2% Hämolyse wird klassifiziert als nicht-hämolytisch, 2 bis 5% als leicht hämolytisch und > 5% als hämolytisch. Die Werte repräsentieren den Mittelwert aus drei Messungen (± Standardabweichung).

Das Polymer **P3** verursacht keine Aggregation von Erythrozyten und zeigt keine Hämolyse im Gegensatz zu **P1** und **P2** (Figur 2).

### b) Bildungsrate und Stabilität der Polyplexbildung

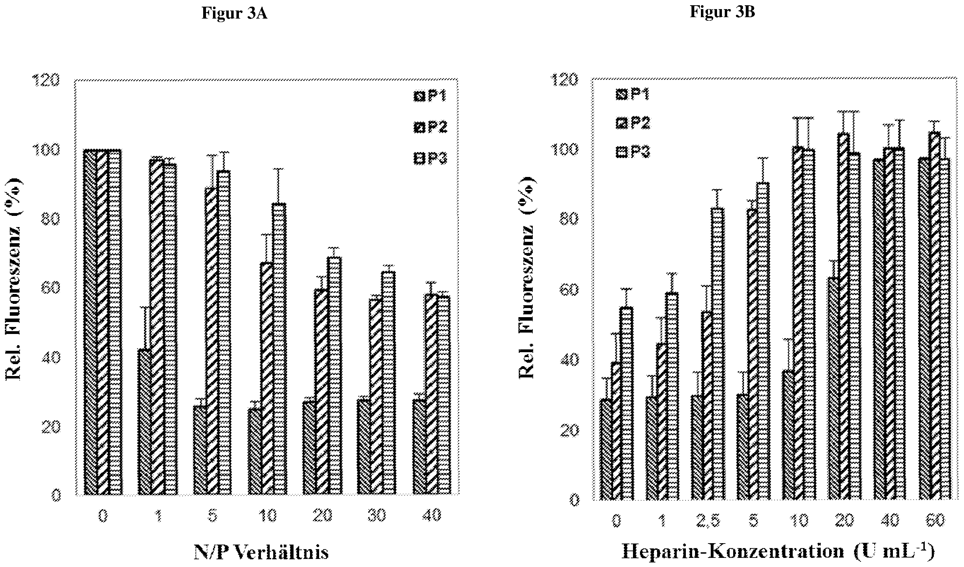

Die Fähigkeit genetisches Material zu komplexieren ist ein zentrales Anliegen des verwendeten, kationischen Polymers. Um dies zu überprüfen, werden verschiedene Verhältnisse (N/P-Verhältnisse) der Summe aller Stickstoff-Atome (N) des kationischen Polymers und der Phosphor-Atome (P) des genetischen Materials getestet. Figur 3A und **3B** zeigt die Polyplex-Formation und Stabilität mit pDNA der Polymere **P1, P2** and **P3.** Figur 3A zeigt hierbei im speziellen die Bindungsaffinität unter angegebenen N/P-Verhältnissen (Ethidiumbromid Quenching-Assay) und Figur 3B zeigt den Dissoziations-Assay der Polyplexe bei einem N/P Verhältnis von 20 unter Verwendung von Heparin (0 to 60 U mL⁻¹). Die Werte spiegeln das Mittel aus drei Messungen wieder ± S.D (n=3).

Das D-Fructose konjugierte Polymer **P3** zeigt stabile Polyplexbildung bei einem N/P Verhältnis >15 und zeigt weiterhin eine zügige Freisetzung des genetischen Materials in Anwesenheit von Heparin (Figur 3).

### c) Größe der Polyplexe

| **Polymer** | **z-Durchschnitt [d/nm]** | **PDI** | **Numerischer Durchschnitt [d/nm]** | **Zetapotential [mV]** |
|---|---|---|---|---|
| **P1** | 217±8 | 0.47 | 71 ± 13 | 24.0 ± 0.4 |
| **P2** | 264 ± 11 | 0.35 | 109 ± 33 | 24.3 ± 1.1 |
| **P3** | 165 ± 1 | 0.26 | 83 ± 29 | 17.6 ± 0.4 |

Die Tabelle zeigt Größe und Zeta-Potential der Polyplexe von **P1** bis **P3** bei N/P 20 im HBG Puffer (gemessen mit Hilfe von dynamischer und elektrophoretischer Lichtstreuung).

### d) Zellaufnahme

Um die Resultate der Zelltoxizitätsstudien zu unterstützen, wurden die Polymere mit verschiedenen Farbstoffen (Cy-5 und Rhodamin-SCN) markiert, mit den erwähnten Zelllinien inkubiert und die Ergebnisse mittels Durchflusszytometrie (FACS) und konfokaler Laserscanning-Mikroskopie (CLSM) evaluiert.

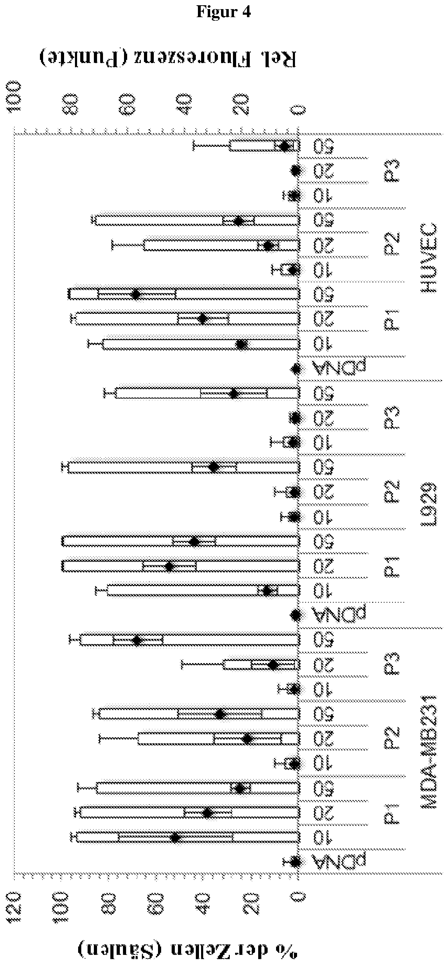

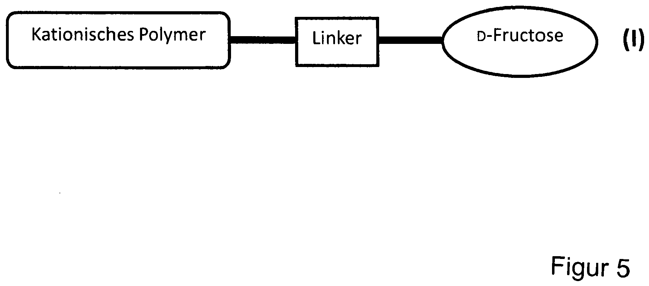

Figur 4 zeigt die Zellaufnahme-Studien. Polyplexe der Polymere **P1** bis **P3** wurden mit YOYO-1 markierter pDNA und L929-, HUVEC- und MDA-MB-231-Zellen inkubiert. Abgebildet ist die relative, mittlere Fluoreszenzintensität (MFI) aller lebenden Zellen verglichen zur pDNA-Kontrolle ohne Polymer (Punkte). Die Werte spiegeln das Mittel aus drei Messungen wieder ± S.D (n=3).

**P1** und **P2** zeigen hierbei eine unspezifische Aufnahme in alle betrachteten Zelllinien (5-60%) bei allen N/P Verhältnissen. **P3** hingegen zeigt eine signifikant erhöhte Aufnahme in die Brustkrebszelllinie MDA-MB-231 für N/P = 50 (60%) im Vergleich zu **P1** und **P2** (20-30%). Des Weiteren zeigt **P3** eine deutlich verringerte Aufnahme in die Nicht-Brustkrebszelllinie L929 (20%) und die menschliche Primärzelllinie HUVEC (5%) für N/P = 50. Der deutliche Unterschied im Uptake-Verhalten in MDA-MB-231 zwischen der unmittelbaren Vorstufe **P2** und dem D-Fructose-konjugierten **P3** unterstreicht eine erfolgreiche Targeting-Funktion des Zucker-Moleküls. Die Säulen in Figur 4 spiegeln den prozentualen Anteil der Zellen wieder, die durch pDNA-Aufnahme überhaupt Fluoreszenz aufweisen.

Diese Ergebnisse wurden auch mittels konfokaler Laserscanning-Mikroskopie der Zellen beobachtet, wenn diese mit den Farbstoff-markierten Polymeren inkubiert wurden. Bei N/P 50 war die Fluoreszenzintensität von **P3** in L929 gering und hoch in MDA-MB-231 Zellen, wogegen Polymere **P1** und **P2** einen reversen Trent aufzeigten. Die Resultate der Aufnahme-Studien in lebenden Zellen sind in Einklang mit den Ergebnissen der Zytotoxizitätsassays und zeigen damit eine Zelltypspezifität des D-Fructose-konjugierten Polymers **P3.**

## Patentansprüche

1. Kationische Polymere mit kovalent gebundener D-Fructose der allgemeinen Formel (I) mit den Bestandteilen:
• a) Kationisches Polymer in Form von makromolekularen Verbindungen aus n Wiederholungseinheiten mit einer oder mehreren positiven Ladungen;
• b) D-Fructose oder mehrere D-Fructose oder Derivate von D-Fructose
• und Linker, wobei der Linker das kationische Polymer mit der D-Fructose oder mit Derivaten von D-Fructose mittels eines beliebigen Alkyl- oder Aryl-Restes, eines beliebigen Alkenyl oder Alkinyl-Restes, eines Ethers, Thioethers oder Amins, eines Ester-, Amid- oder eines anderen Carbonsäure-Derivats, eines Heterozyklus`, eines Disulfids, eines Imins bzw. eines Imids verknüpft,
**dadurch gekennzeichnet, dass** die Fructose oder mehrere D-Fructose oder D-Fructose-Derivate in offenkettiger, furanoider oder pyranoider Struktur über eines seiner fünf möglichen Kohlenstoffatome in C1-, C3-, C4-, C5- oder C6-Position nicht- glykosidisch mit dem Linker verknüpft ist.

2. Kationische Polymere mit kovalent gebundener D-Fructose gemäß Anspruch 1, **dadurch gekennzeichnet, dass** sie funktionelle Gruppen aufweisen, welche unter entsprechenden Bedingungen positive Ladungen aufweisen.

3. Kationische Polymere mit kovalent gebundener D-Fructose gemäß mindestens einem der Ansprüche 1 bis 2, **dadurch gekennzeichnet, dass** sie funktionelle Gruppen, welche positive Ladungen tragen, an verschiedenen Positionen einmal oder mehrfach im Polymer enthalten.

4. Kationische Polymere mit kovalent gebundener D-Fructose gemäß mindestens einem der Ansprüche 1 bis 3, **dadurch gekennzeichnet, dass** sie sowohl als Homo- oder als Copolymer ausgebildet sind.

5. Kationische Polymere mit kovalent gebundener D-Fructose gemäß mindestens einem der Ansprüche 1 bis 4, **dadurch gekennzeichnet, dass** sie linear oder verzweigt sind, wobei letztere Form Sterne, Bürsten und Kämme einschließt.

6. Kationische Polymere mit kovalent gebundener D-Fructose gemäß mindestens einem der Ansprüche 1 bis 5, **dadurch gekennzeichnet, dass** eine oder mehrere D-Fructose-Reste an einzelne, mehrere oder alle Wiederholungseinheiten des kationischen Polymers über den Linker gebunden sind.

7. Kationische Polymere mit kovalent gebundener D-Fructose gemäß mindestens einem der Ansprüche 1 bis 6, **dadurch gekennzeichnet, dass** die D-Fructose neben freien OH-Gruppen weitere Substituenten an den Kohlenstoffatomen 1, 2, 3, 4, 5 und / oder 6 trägt.

8. Kationische Polymere mit kovalent gebundener D-Fructose gemäß mindestens einem der Ansprüche 1 bis 7, **dadurch gekennzeichnet, dass** ein biologisch aktives Material aus der Gruppe der Nucleinsäuren elektrostatisch und/oder kovalent gebunden ist.

9. Kationische Polymere mit kovalent gebundener D-Fructose gemäß Anspruch 8, **dadurch gekennzeichnet, dass** die gebundene Nucleinsäure aus der Gruppe von DNA, RNA, einem Ribosom und/oder einem DNA-RNA-Hybrid ist und dabei doppelsträngig und/oder einzelsträngig vorliegt.

10. Verwendung des kationischen Polymers mit kovalent gebundener D-Fructose gemäß mindestens einem der Ansprüche 1 bis 9 zum Transport und der Abgabe eines biologisch aktiven Materials in eine lebende Zelle.

11. Verwendung des kationischen Polymers mit kovalent gebundener D-Fructose gemäß mindestens einem der Ansprüche 1 bis 9 zur selektiven Abtötung bestimmter Zellarten.

## Claims

1. Cationic polymers with covalently bound D-fructose of the general formula (I) with the constituents:
- a) cationic polymer in the form of macromolecular compounds of n repeating units having one or more positive charges;
- b) D-fructose or a plurality of D-fructoses or derivatives of D-fructose
- and linker, wherein the linker links the cationic polymer to the D-fructose or to derivatives of D-fructose by means of any alkyl or aryl radical, any alkenyl or alkynyl radical, an ether, thioether or amine, an ester, amide or other carboxylic acid derivative, a heterocycle, a disulphide, an imine or an imide,
**characterised in that** the fructose or several D-fructose or D-fructose derivatives in open-chain, furanoid or pyranoid structure is non-glycosidically linked to the linker via one of its five possible carbon atoms in C1, C3, C4, C5 or C6 position.

2. Cationic polymers with covalently bonded D-fructose according to claim 1, **characterised in that** they have functional groups which have positive charges under corresponding conditions.

3. Cationic polymers with covalently bonded D-fructose according to at least one of claims 1 to 2, **characterised in that** they contain functional groups, which carry positive charges, at different positions once or several times in the polymer.

4. Cationic polymers with covalently bonded D-fructose according to at least one of claims 1 to 3, **characterized in that** they are formed both as homopolymers and as copolymers.

5. Cationic polymers with covalently bound D-fructose according to at least one of claims 1 to 4, **characterised in that** they are linear or branched, the latter form including stars, brushes and combs.

6. Cationic polymers with covalently bound D-fructose according to at least one of claims 1 to 5, **characterized in that** one or more D-fructose residues are bound to single, multiple or all repeating units of the cationic polymer via the linker.

7. Cationic polymers with covalently bonded D-fructose according to at least one of claims 1 to 6, **characterized in that** the D-fructose carries, in addition to free OH groups, further substituents on carbon atoms 1, 2, 3, 4, 5 and/or 6.

8. Cationic polymers with covalently bonded D-fructose according to at least one of claims 1 to 7, **characterised in that** a biologically active material from the group of nucleic acids is electrostatically and/or covalently bonded.

9. Cationic polymers with covalently bound D-fructose according to claim 8, **characterized in that** the bound nucleic acid is from the group of DNA, RNA, a ribosome and/or a DNA-RNA hybrid and is present in double-stranded and/or single-stranded form.

10. Use of the cationic polymer comprising covalently bound D-fructose according to at least one of claims 1 to 9 for transporting and delivering a biologically active material into a living cell.

11. Use of the cationic polymer with covalently bound D-fructose according to at least one of claims 1 to 9 for selectively killing specific cell types.

## Revendications

1. Polymères cationiques contenant du D-fructose lié par covalence, de formule générale (I) ayant les composants suivants :
- a) Polymère cationique sous forme de composés macromoléculaires de n unités de répétition avec une ou plusieurs charges positives,
- b) du D-fructose ou plusieurs D-fructoses ou des dérivés de D-fructose
- et des agents de liaison, l'agent de liaison liant le polymère cationique au D-fructose ou à des dérivés du D-fructose au moyen de n'importe quel radical alkyle ou aryle, n'importe quel radical alcényle ou alcynyle, un éther, un thioéther ou une amine, un ester, un amide ou un autre dérivé d'acide carboxylique, un hétérocycle, un disulfure, une imine ou un imide, respectivement,
**caractérisé en ce que** le fructose ou plusieurs D-fructoses ou dérivés de D-fructose de structure à chaîne ouverte, furanoïde ou pyranoïde sont liés de manière non glycosidique au lieur par l'un de ses cinq atomes de carbone possibles en position C1, C3, C4, C5 ou C6.

2. Polymères cationiques avec du D-fructose lié de manière covalente selon la revendication 1, **caractérisés en ce qu'**ils présentent des groupes fonctionnels qui, dans des conditions appropriées, présentent des charges positives.

3. Polymères cationiques avec du D-fructose lié de manière covalente selon au moins l'une des revendications 1 à 2, **caractérisés en ce qu'**ils contiennent des groupes fonctionnels qui portent des charges positives à différentes positions, une ou plusieurs fois dans le polymère.

4. Polymères cationiques avec du D-fructose lié de manière covalente selon au moins l'une des revendications 1 à 3, **caractérisés en ce qu'**ils sont formés à la fois comme homopolymère et comme copolymère.

5. Polymères cationiques à D-fructose lié de manière covalente selon au moins l'une des revendications 1 à 4, **caractérisés en ce qu'**ils sont linéaires ou ramifiés, cette dernière forme incluant les étoiles, les brosses et les peignes.

6. Polymères cationiques comprenant du D-fructose lié de manière covalente selon au moins l'une des revendications 1 à 5, **caractérisés en ce qu'**un ou plusieurs résidus de D-fructose sont liés à un, plusieurs ou tous les motifs répétitifs du polymère cationique via le lieur.

7. Polymères cationiques avec du D-fructose lié de manière covalente selon au moins l'une des revendications 1 à 6, **caractérisés en ce que** le D-fructose porte, outre des groupes OH libres, d'autres substituants sur les atomes de carbone 1, 2, 3, 4, 5 et/ou 6.

8. Polymères cationiques avec du D-fructose lié de manière covalente selon au moins l'une des revendications 1 à 7, **caractérisés en ce qu'**une matière biologiquement active du groupe des acides nucléiques est liée de manière électrostatique et/ou covalente.

9. Polymères cationiques avec du D-fructose lié de manière covalente selon la revendication 8, **caractérisés en ce que** l'acide nucléique lié est choisi dans le groupe de l'ADN, de l'ARN, d'un ribosome et/ou d'un hybride ADN-ARN et se présente alors sous forme double brin et/ou simple brin.

10. Utilisation du polymère cationique contenant du D-fructose lié de manière covalente selon au moins l'une des revendications 1 à 9 pour le transport et la libération d'un matériau biologiquement actif dans une cellule vivante.

11. Utilisation du polymère cationique contenant du D-fructose lié de manière covalente selon au moins une des revendications 1 à 9 pour tuer sélectivement certains types de cellules.
